# EUROPEAN PATENT APPLICATION

(11) **EP 4 023 152 A1**
(43) Date of publication of application: **06.07.2022**
(21) Application number: 21150189.5
(22) Date of filing: 05.01.2021
(51) Int. Cl.: A61B 5/055, A61B 5/00, G16H 50/00

(54) **SYSTEM AND METHOD OF PATIENT PROFILE CREATION**

(71) Applicant: Koninklijke Philips N.V., 5656 AG Eindhoven (NL)
(72) Inventor: SREENIVASAN, Rithesh, 5656 AE Eindhoven (NL); BUSSA, Nagaraju, 5656 AE Eindhoven (NL); PALANISAMY, Krishnamoorthy, 5656 AE Eindhoven (NL); VOGTMEIER, Gereon, 5656 AE Eindhoven (NL); JOHNSON, Mark Thomas, 5656 AE Eindhoven (NL); SISODIA, Rajendra Singh, 5656 AE Eindhoven (NL); WIESS, Steffen, 5656 AE Eindhoven (NL); LEUSSLER, Christoph Günther, 5656 AE Eindhoven (NL)
(74) Representative: Philips Intellectual Property & Standards

(57) **Abstract**

In order to improve workflow efficiency and/or patient experience during a scan procedure, a system is proposed to provide - prior to arrival at the hospital - an indication of the suitability of a patient to be assigned to a specific level of autonomy in a medical scanning procedure. The system comprises a scan simulation module, a patient monitoring module, and a patient profile generation module. The scan simulation module comprises one or more sensory stimulation devices configured to apply at least one sensory stimulus over a patient to simulate a scan environment that may be experienced by a patient during a scan procedure. The patient monitoring module comprises one or more sensors configured to acquire data of the patient in the simulated scan environment. The patient profile generation module is configured to determine a state of anxiety of the patient based on the acquired data and to create a patient profile comprising the determined state of anxiety of the patient in the simulated scan environment.

## Description

### FIELD OF THE INVENTION

The present invention relates to medical imaging, and in particular to a system for patient profile creation, a method for patient profile creation, a computer program element, and a computer readable medium.

### BACKGROUND OF THE INVENTION

In the use of magnetic resonance imaging (MRI) as a diagnostic procedure, the problem of anxiety in MRI patients and other claustrophobia-related symptoms has become more and more pronounced. The main source of anxiety is the need to remain lying down in the narrow gantry space during the examination. Other factors, such as a concern about the examination result, high noise, vibration, isolation from the environment, or immobilisation of the head during brain scans, may further increase the level of anxiety in the patient. For these reasons, the reported incidence of premature termination or failure of the MRI examination ranges between 0.5% and 14.5%, and the reported incidence of anxiety-related reactions during MRI reaches 37%.

Many patients referred for positron emission tomography (PET) suffer from anxiety, possibly affecting the workflow and patient experience. In addition, patient anxiety may affect image quality through uptake of 18F-FDG in muscles or brown adipose tissue (BAT).

### SUMMARY OF THE INVENTION

There may be a need to improve workflow efficiency and/or patient experience during a scan procedure.

The object of the present invention is solved by the subject-matter of the independent claims, wherein further embodiments are incorporated in the dependent claims. It should be noted that the following described aspects of the invention apply also for the system for patient profile creation, the method for patient profile creation, the computer program element, and the computer readable medium.

According to a first aspect of the present invention, there is provided a system for patient profile creation. The system comprises a scan simulation module, a patient monitoring module, and a patient profile generation module. The scan simulation module comprises one or more sensory stimulation devices configured to apply at least one sensory stimulus over a patient to simulate a scan environment that may be experienced by a patient during a scan procedure. The patient monitoring module comprises one or more sensors configured to acquire data of the patient in the simulated scan environment. The patient profile generation module is configured to determine a state of anxiety of the patient based on the acquired data and to create a patient profile comprising the determined state of anxiety of the patient in the simulated scan environment.

In other words, a system of patient profile creation is proposed using simulated scan procedures along with sensors to determine a state of anxiety of the patient in the simulated scan procedure. In order to more fully immerse the patient in the scan environment, scan simulation module may utilize any or all human senses. Sight may be utilized through the use of different perspectives such as through the user's sight, from above or below, or from a perspective view, e.g. via a head-mounted display. Sound, such polyphonic, may be used to recreate spatial perception of e.g. noise. The vibration may be simulated through the tactile sensor devices such as a vibrating device, pressure generating devices, and the like. The scan simulation module may provide a virtual environment of a scan and the feeling during the scan procedure to the patient in order to measure patient's reaction and understand if the patient could act in a compliant way with the proposed scanner/scan. Otherwise, a more compatible scanner (e.g. open MRI, large bore, etc.) and scan protocol may be selected to increase the chance for a successful imaging procedure.

The patient monitoring module may comprise a camera to acquire image data of the patient. Heart rate, respiration rate, motion, facial expression, and/or pupil size may be derived from the image data. Alternatively or additionally, physiological sensors may be used to acquire physiological data of the patient. For example, the physiology sensor may be a temperature sensor to measure the patient's skin temperature, or a conductivity sensor (Ohm meter) to measure the electrical conductivity of the patient's skin.

The patient profile generation module may comprise a computing device that determines a state of anxiety of the patient based on the acquired data and to create a patient profile comprising the determined state of anxiety of the patient in the simulated scan environment. This patent profile may be used to modify the scan stages or measure safety thresholds, for each of the patients, assess the level of autonomy the patient can be subjected to during the scan procedure and use the profile for making decisions during autonomous scanning.

In some examples, the scan simulation module, the patient monitoring module, and the patient profile generation module may be built-in units of a head-mounted display (HDM). For example, the HMD is a virtual reality enabled HMD, which may have a built-in camera for monitoring the pupil size of the patient and a processor for determining a stress level based on the monitored pupil size of the patient to create the patient profile.

In some examples, the scan simulation module, the patient monitoring module, and the patient profile generation module may be individual devices. For example, the scan simulation module may be an HMD. The patient monitoring module may comprise wearable sensors. The patient profile generation module may be a tablet computer.

In some examples, the scan simulation module may assign the patient to a scanning procedure with a specific level of autonomy in combination with an appropriate matching equipment setting for this patient. The specific level of autonomy may specify whether it is autonomic imaging or would require presence of staff support.

In some examples, the proposed system may provide training for undergoing the prescribed scan at home and reduce the anxiety levels and measure the limits accepted by the patient.

In some examples, the system may comprise a personalized module to create augmented reality (AR)/ virtual reality (VR) training materials that capture the digitized autonomous environment and AR/VR scan sequence adapted to the person specific education and instruction content.

In some examples, the patent profile generation module may analyze a patient behavior during the simulated scan procedure with the help of biological and/or wearable sensors and update the scan sequence for a better patient co-operation.

In some examples, the system may comprise a recommendation system for determining additional support system, such as breathing support and staff support at specific scan workflow step, which will be required based on assessment of the simulated scan procedure. The recommendation system may recommend a suitable scanner type, e.g. open or closed-bore, 1.5T or 3T scanner, based on patient behavior during the simulated scan procedure depending on susceptibility to claustrophobia, noise, general anxiety, and/or supported scan duration. The recommendation system may recommend a radiofrequency coil based on the anxiety and stress level of the patient. The recommendation system may recommend scan sequence changes for a better patient co-operation when the patient arrives for the real scan.

According to an embodiment of the present invention, the patient profile generation module is further configured to determine one or more of the following parameters based on the acquired data: a reason of anxiety and/or a time instant at which the patient has a high level of anxiety during the scan procedure. The patient profile further comprises the one or more determined parameters.

Main reasons of anxiety may be determined to find whether it is caused by claustrophobia, high noise, and/or related long scan duration. This may be done by comparing the anxiety and stress levels in different scan environments. These scan environments may be different from each other in a scanner type (e.g. open or closed-bore scanner), a level of autonomy of the scan procedure (e.g. autonomous or non-autonomous scanner), a machine specification of a scanner (e.g. bore size, field strength, etc.), and/or a scan protocol (e.g. different combinations of e.g. various MRI sequences).

The time instant at which the patient has a high level of anxiety during the scan procedure indicates when the patient is highly stressed. At this time instant, a staff support and/or a support device (e.g. external breath hold assistance, patient positioning device, etc.) may be required.

According to an embodiment of the present invention, the patient monitoring module comprises a sensor configured to monitor a behavior of the patient during the simulated scan procedure. The patient profile generation module is configured to determine whether the patient acts in a compliant way with the simulated scan environment based on the monitored behavior of the patient.

For example, a camera may be used to monitor the motion of the patient to determine the ability of the patient to stay still during the simulated scan.

For example, a respiratory rate sensor may be used to determine the capacity of patient breath-hold.

According to an embodiment of the present invention, the scan simulation module is configured to modify the at least one applied sensory stimulus to simulate a plurality of scan environments that may be experienced by the patient. The patient monitoring module is configured to acquire data of the patient in the plurality of simulated scan environments. The created patient profile comprises the determined state of anxiety of the patient in the plurality of simulated scan environments.

By comparing the anxiety and stress levels of the patient in different simulated scan environments, it is possible to find whether the anxiety of the patient is caused by claustrophobia, high noise, and/or related long scan duration.

According to an embodiment of the present invention, the plurality of scan environments are different from each other in one or more of a scanner type to be used, a level of autonomy of the scan procedure, a machine specification of a scanner, and a scan protocol.

According to an embodiment of the present invention, the scan simulation module comprises one or more of the following sensory stimulation devices: a visual stimulation device, a tactile stimulation device, and an auditory stimulation device.

The visual stimulation device may be used to provide a virtual environment of a scan. An example of the visual stimulation device is an HDM.

The tactile stimulation device and the auditory stimulation devices may be used to provide the feeling during the scan procedure to the patient. For example, the tactile stimulation device, such as a vibration device, pressure generating devices, and the like, may be used to simulate vibration during the scan.

The auditory stimulation device may be used to simulate a noise environment of the scan procedure according to e.g. the field strength of the scanner.

According to an embodiment of the present invention, the scan simulation module is configured to create an immersive environment.

Immersive environments are simulations that fill the patient's visual field, giving the sensation of physical presence.

According to an embodiment of the present invention, the patient monitoring module comprises one or more of the following sensors a camera to acquire image data of the patient and a physiology sensor configured to acquire physiological data of the patient.

From the image data, several parameters of the imaged patient may be derived, such as heart rate, respiration rate, motion, facial expression, and pupil size.

For example, the physiology sensor may be a temperature sensor to measure the patient's skin temperature, or a conductivity sensor to measure the electrical conductivity of the patient's skin. Other examples of the physiology sensors may include e.g. electrocardiography (ECG) sensor, heart rate sensor, blood pressure sensor, and galvanic skin sensor.

According to an embodiment of the present invention, the system further comprises a patient training module configured to provide instructions to guide the patient through the scan procedure, and/or to educate the patient to identify a relevant set of feedback communication methods to provide the feedback during the scan procedure.

In an example, the instructions may specify how breath the patient should inhale for breath hold in breath hold imaging e.g. in MRI.

In an example, the instructions may indicate the posture on how and where the patient should stand, such as standing, standing holding 8 kg, standing 45 flexion, seated 45 flexion, seated upright, seated 45 extension, and supine.

In an example, the instructions may specify patient's positioning (when and how to position).

In an example, the instructions may comprise safety instructions.

These instructions and education materials may be demonstrated in an AR/VR environment. For example, these contents may be synthesized with the basic MRI sequence using the basic visemes super imposed on a programmable 3D model.

According to an embodiment of the present invention, the system further comprises a recommendation module configured to generate a personalized recommendation for an actual scan procedure for the patient based on the created patient profile.

According to an embodiment of the present invention, the personalized recommendation comprises one or more of: selection of a scan protocol, selection of a scanner type, selection of a relaxation technique for the patient, indication of the suitability of a patient to be assigned to a specific level of autonomy in a medical scanning procedure, selection of a support device required for an autonomous scan, selection of a parameter for a therapy system, and provision of an augmented reality, AR, based patient guidance for an actual patient scan process.

In an example, the recommendation module may determine scan sequence changes, i.e. a change of scan protocol, based on feasibility analysis for the simulated scan procedure. For example, the recommendation module may analyse the patient's behaviour during the simulated scan procedure with the help of biological and/or wearable sensors and update the scan sequence for a better patient co-operation.

In an example, the recommendation system may recommend a suitable scanner type, e.g. open or closed-bore, 1.5T or 3T scanner, based on patient behavior during the simulated scan procedure depending on susceptibility to claustrophobia, noise, general anxiety, and/or supported scan duration.

In an example, the recommendation system may recommend relaxation techniques (e.g. music, videos, breathing techniques) followed by a second evaluation to see if the patient improves.

In an example, the recommendation system may evaluate if the patient is a right choice for autonomous imaging or would require presence of support staff according to a compliance of the behavior of the patient (e.g. the ability to stay still during the simulated scan procedure, the capability of breath hold in breath hold imaging, and the like) with the simulated scan environment.

In an example, the recommendation system may evaluate if the patient is a right choice for autonomous imaging or would require presence of support staff based on the level of anxiety of the patient during the simulated scan procedure. This may be implemented in a scenario where hospitals have a mix of autonomous and non-autonomous scans.

In an example, the recommendation system may determine additional support system, such as breathing support and staff support at specific scan workflow step, which will be required based on assessment of the simulated scan procedure.

In an example, the recommendation system may recommend an RF coil based on the anxiety and stress level of the patient. The recommendation system may recommend scan sequence changes for a better patient co-operation when the patient arrives for the real scan.

In an example, the recommendation system may recommend a therapy mask type, selection and adaptation of therapy settings for a therapy system like MR-Linac/MRHifu based on the created patient profile.

In an example, the recommendation module may provide an AR based patient guidance during the actual patient scan process based on a virtual environment scan process feedback.

According to an embodiment of the present invention, the system is a head-mounted system.

According to a second aspect of the present invention, there is provided a method for patient profile creation. The method comprises:
- applying at least one sensory stimulus over a patient to simulate a scan environment that may be experienced by a patient during a scan procedure;
- acquiring data of the patient in the simulated scan environment; and
- determining a state of anxiety of the patient based on the acquired data and creating a patient profile comprising the determined state of anxiety of the patient in the simulated scan environment.

According to another aspect of the present invention, there is provided a computer program element for controlling a system according to the first aspect and any associated example, which when being executed by a processor is configured to carry out the method according to the second aspect.

According to another aspect of the present invention, there is provided a computer readable medium comprising the computer program element.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the invention will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.
Fig. 1 schematically shows an example of a system for creating a patient profile.
Fig. 2 schematically shows a further example of a system for creating a patient profile.
Fig. 3 shows a flowchart of a method for creating a patient profile.

### DETAILED DESCRIPTION OF EMBODIMENTS

In the use of e.g. MRI, computed tomography (CT) and PET as a diagnostic procedure, the problem of anxiety in patients and other claustrophobia-related symptoms have become more and more pronounced. This may adversely affect the workflow efficiency and patient experience.

In order to improve the workflow efficiency and/or the patient experience, a system is proposed to create - prior to arrival at the hospital - a patient profile, which is usable to generate a personalized recommendation for an actual scan procedure for the patient to reduce the anxiety in patients.

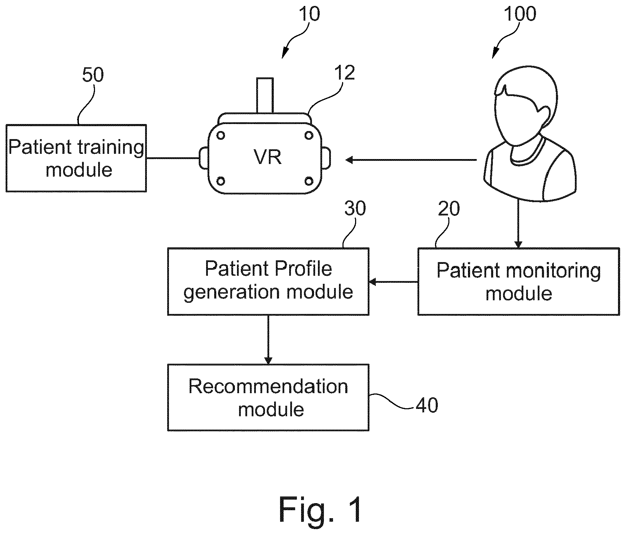

Fig. 1 illustrates an example of a system 100 for patient profile creation. In the following, the approach is described in relation with MRI scans. Although the following detailed description is described using application to MRI scans for the purposes of illustration, anyone of ordinary skill in the art will appreciate that the system and method described above and below can be adapted to other imaging modalities, e.g. CT or PET. Accordingly, the following described examples are set forth without any loss of generality to, and without imposing limitations upon, the claimed invention.

In the example of Fig. 1, the system 100 comprises a scan simulation module 10, a patient monitoring module 20, and a patient profile generation module 30. Optionally, as shown in Fig. 1, the system 100 may further comprise a recommendation module 40 and a patient training module 50.

The scan simulation module 10 comprises one or more sensory stimulation devices configured to apply at least one sensory stimulus over a patient to simulate a scan environment that may be experienced by a patient during a scan procedure.

In the example shown in Fig. 1, the scan simulation module 10 comprises a visual stimulation device 12 to simulate a virtual environment of the scan procedure. The exemplary visual stimulation device 12 in Fig. 1 is a head-mounted display (HMD) that immerses the patient in the scan environment. For example, the exemplary HMD may include two small high resolution OLED or LCD monitors that provide separate images for each eye for stereoscopic graphics rendering a 3D virtual world, and positional and rotational real-time head tracking for six degrees of movement.

In order to more fully immerse the patient in the scan environment, the scan simulation module 10 may simulate further unpleasant aspects of the examination such as scanner noise and vibration.

In an example, the scan simulation module 10 may comprise a tactile stimulation device (not shown) to simulate vibration during the scan. The tactile stimulation device may be a vibrating device that is touching or very close to the patient and in response to a control signal, vibrates e.g. at predetermined vibration strength and frequency and at predetermined timings. In an example, piezoelectric actuators may be employed to produce vibrations. In another example, focused ultrasound beams may be used to create a localized sense of pressure on the skin without touching the patient.

The scan simulation module 10 may further comprise an auditory stimulation device (not shown) to simulate a noise environment of the scan procedure. For example, the HMD in Fig. 1 may further comprise a binaural audio system to create noise levels the patient may be experienced during imaging.

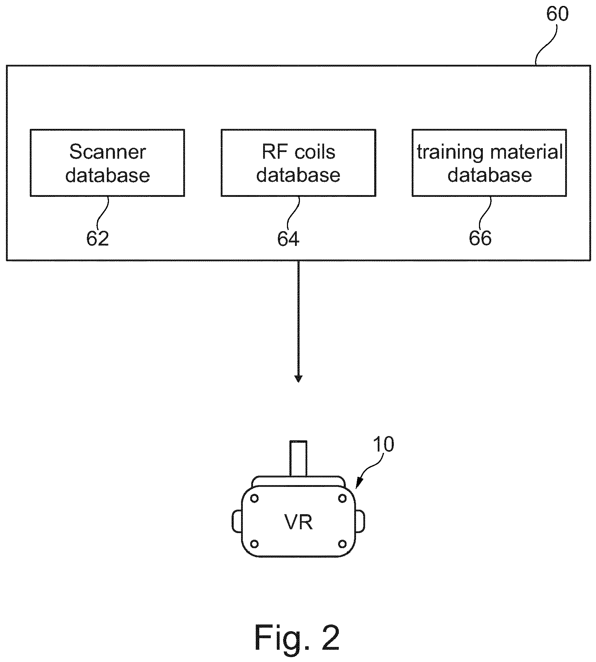

As shown in Fig. 2, the scan simulation module 10, such as the HMD shown in Fig. 1, may obtain virtual reality contents from a virtual reality content server 60 to provide a simulated scan environment that may be experienced by the patient during a scan procedure. The virtual reality contents may have imaging and virtual reality data that is for rending the scan environment in the virtual reality enabled scan simulation module.

In the example of Fig. 2, the virtual reality content server 60 may have a scanner database 62. The scanner database 62 may comprise three-dimensional (3D) models of a plurality of scanners. These 3D models may be described using a virtual reality modeling language (VRML), which is based on certain types of polygons defined by a set of parameters. One or more of the VRML 3D models of the scanners may be downloaded to the scan simulation module 10 to render the scene locally. These 3D models of the scanners may be programmable, such that e.g. instructions to guide the patient through the scan procedure, may be combined and synthesized with basis sequence e.g. using the basis visemes superimposed on the programmable 3D models.

For example, the scanner database 62 may comprise 3D models of open and closed-bore scanners. For example, the closed MRI features a capsule-like space the patient would lie in to have high-quality images taken. The shape and dimensions of the scans are tightly enclosed, which can cause some patients to feel nervous. Because it takes a few minutes to complete a full scan, it increases the claustrophobia risk, and panic attacks may develop sometimes. Rather than being an enclosed capsule, the open MRI uses a magnetic bottom and top and all four sides are open, reducing the risk of panic attacks and claustrophobia exponentially, allowing the patient to make use of an MRI to diagnose the condition accurately.

Besides the 3D models of different scanners, the scanner database 62 may further comprise a machine specification of each scanner. In an example, the machine specification of the scanner may specify the field strength of the scanner. Taking MRI as an example, the vibration of gradient coils due to rapidly switched electrical currents produces sound waves during routine MR imaging. Some sequences (especially echo-planar imaging) generate sound pressures as high as 110-130 dB. In general, 3T scanners are noisier than those operating at 1.5T. Acoustic noise at the higher end of this range may cause temporary or permanent hearing loss. Even noise of lower intensity may cause anxiety or distress in patients, especially infants, young children, and the elderly. This may manifest as patient motion artifacts and degraded image quality. In an example, the machine specification of the scanner may specify whether the scanner is an autonomous scanner or a non-autonomous scanner. An autonomous imaging would be possible if the patient does not experience severe anxiety or stress during the simulated scan procedure. In another example, the machine specification of the scanner may further specify the bore size of the scanner.

The virtual reality content server 60 may further comprise an RF coil database 64, which may comprise 3D models of a plurality of radiofrequency (RF) coils. In MR scanners signal reception is achieved by using either transmit-receive or receive-only RF coils. The RF coils are in close contact to the patient body and face, which may considerably provoke stress and anxiety. In addition to the 3D models of the RF coils, the RF coil database 64 may further comprise a specification of each RF coil. For example, the specification of each RF coil may specify size, weight, diameter, space, flexibility, and/or cable or wireless of the RF coil.

The virtual reality content server 60 may further comprise a training material database 66. The training material database 66 may comprise instructions to guide the patient through the scan procedure. For example, the instructions may indicate how breath the patient should inhale for breath hold. For example, the instructions may indicate posture on how and where the patient should stand, positioning (when and how to position), safety instructions, etc. for different anatomies, such as the head/neck area, the spine and the abdomen. The training material database 66 may also comprise instructions to educate the patient to identify a relevant set of feedback communication methods to provide the feedback during the scan procedure. That is, according to the instructions the patient is able to identify what are different ways of feedbacks at different time instants (if needed) to be given to the system during the scan so that subsequent appropriate actions may be performed in a completely autonomous settings.

In order to simulate the scan procedure, a 3D model of a scanner may be selected from the scanner database. Every scan consists of a set of sequences. These basic sequences may be defined. Then based on a scan protocol, these basic sequences can be combined to form a scan. The AR/VR scenes can thus be created for these basic sequences and then based on the selected scanner and the scan protocol to re-create the entire scan in AR/VR using composition of basic scenes. Optionally, 3D models of RF coils may also be added to the basis sequences to select an appropriate coil that fits to profile (stress and anxiety level) of the patient.

Turning back to Fig. 1, the optional patient training module 50 is configured to provide instructions to guide the patient through the scan procedure. Alternatively or additionally, the optional patient training module 50 is configured to educate the patient to identify a relevant set of feedback communication methods to provide the feedback during the scan procedure. The patient training module 50 may retrieve these contents from the training material database 66 based on the scan description. The retrieved contents, such as instructions to guide the patient through the scan procedure, may be combined and synthesized with the basis sequence e.g. using the basis visemes superimposed on a programmable 3D model.

The patient monitoring module 20 further comprises one or more sensors configured to acquire data of the patient in the simulated scan environment.

In an example, the patient monitoring module 20 may comprise a camera to acquire image data of the patient. From the image information, several parameters of the imaged patient may be derived, such as heart rate, respiration rate, motion, facial expression, and pupil size (diameter).

In an example, the patient monitoring module 20 may comprise a physiological sensor configured to acquire physiological data of the patient. For example, the physiology sensor may be a temperature sensor to measure the patient's skin temperature, or a conductivity sensor to measure the electrical conductivity of the patient's skin. Other examples of the physiological sensors may include e.g. electrocardiography (ECG) sensor, heart rate sensor, blood pressure sensor, and galvanic skin sensor.

The acquired data of the patient may be recorded as aligned as per scan sequence for estimating the anxiety and stress level and for non-compliance measurements.

The output of the patient monitoring module 20 is analysed by the patient profile generation module 30. The patient profile generation module 30 is configured to determine a state of anxiety of the patient based on the acquired data and to create a patient profile comprising the determined state of anxiety of the patient in the simulated scan environment. The patient profile may be usable for generating a personalized recommendation for an actual scan procedure for the patient, which will be explained below in more detail with respect to the recommendation module 40.

As an example, the patient profile generation module 30 may include an artificial intelligence (AI) module using a pre-trained AI model for determining the patient's behaviour and physical condition during the simulated scan procedure. The AI model may be pre-trained using the known ground truth samples that can be obtained by feedback from the patient as labels. The algorithm may be a combination of a machine learning approach for the estimation of the current stress level, such as support vector machine (SVM), convolutional neural network (CNN), etc., and a machine learning approach for predicting the development of the stress level during the next few minutes, such as recurrent neural network (RNN) or long short-term memory (LSTM).

The patient psychological and/or biometric condition may be continuously estimating the variations in the patient's vital parameters, such as oxygen saturation and heart rate. The stress level may be determined by analysing the facial expressions using the sequence of images obtained from one or multiple cameras in the simulated scan procedure using the pre-trained AI model. Further, the patient profile generation module 30 may also use the output of the pre-trained AI model as one of the input features for predicting the development of the stress level.

The stress level may be assessed by focusing on the output of specific vital parameter sensors. The stress level may be registered as the sum of the peak intensities or more accurately the sum of the rising edges of the peaks above background level taken as a running average over a period of time of a few minutes. The periodicity of the heart rate variation of the patient may be used instead, or together, with the sum of the peak intensities, whereby a stronger periodicity is associated with a more relaxed patient. If the heart rate variation is both periodic and furthermore synchronous with the breathing rate (e.g. measured by a third vital parameter sensor) of the patient, the patient may be considered to be even more relaxed.

Based on the anxiety and stress levels measured during the simulated scan procedure, the patient may be classified into various categories of anxiety and stress. For example, the anxiety and stress categories may include mild, moderate and severe.

Optionally, the patient profile generation module 30 may be further configured to determine a time instant at which the patient has a high level of anxiety during the scan procedure. In other words, the patient profile generation module 30 may determine when the patient is highly stressed during the simulated scan procedure, and this may be a time instant that requires additional staff support or additional support system (e.g. external breath hold assistance).

In order to find the main reasons of anxiety, such as claustrophobia, high noise, or related long scan duration, the scan simulation module 10 may be configured to modify the at least one applied sensory stimulus to simulate a plurality of scan environments that may be experienced by the patient. The plurality of scan environments may be different from each other in one or more of wherein the plurality of scan environments are different from each other in one or more of a scanner type to be used (e.g. open or closed-bore scanner), a level of autonomy of the scan procedure (e.g. autonomous or non-autonomous), a machine specification of a scanner (e.g. field strength of 1.5T or 3T), and a scan protocol (e.g. different combinations of basic sequences).

In an example, a scanner type to be used may be changed. The scan simulation module 10 may simulate a scan environment of open and closed-bore scanners to determine whether the main reason of anxiety is claustrophobia.

In an example, the scan simulation module 10 may simulate a scan environment of scanners with field strength of 1.5T and 3T to determine whether the main reason of anxiety is noise.

By comparing respective VR/AR situations during the simulated scan procedures, the reasons of anxiety can be determined.

Optionally, the patient monitoring module 20 may comprise a sensor configured to monitor a behavior of the patient during the simulated scan procedure. The patient profile generation module 30 may be configured to determine whether the patient acts in a compliant way with the simulated scan environment based on the monitored behavior of the patient. In an example, the patient monitoring module 20 may comprise a camera to monitor the motion of the patient to determine the ability of the patient to stay still during the simulated scan. This is because many patients may find it difficult to keep their arm and legs still e.g. during an MR examination. Furthermore, the patient may have the urge to release stress or anxiety via body movements. However, only up to a certain magnitude of displacement, these types of motion can be corrected by the MR itself using prospective motion compensation techniques. In an example, the patient monitoring 20 may comprise a respiratory rate sensor to determine the capacity of patient breath-hold. This information may be usable for making decisions during autonomous scanning.

The optional recommendation module 40 is configured to generate a personalized recommendation for an actual scan procedure for the patient based on the created patient profile to reduce the anxiety of the patient during an actual patient scan process.

In an example, the recommendation module 40 may be configured to determine scan sequence changes, i.e. a change of scan protocol, based on feasibility analysis for the simulated scan procedure. For example, the recommendation module may analyse the patient's behaviour during the simulated scan procedure with the help of biological and/or wearable sensors and update the scan sequence for a better patient co-operation.

In an example, the recommendation module 40 may be configured to recommend a scanner type depending the reason(s) of anxiety in the patient profile. For example, patients that are more susceptible to claustrophobia may be scheduled for an open bore scanner. For example, patients that are more susceptible to noise may be scheduled for a 1.5T scanner. For example, patients that are less suited for long scans may be scheduled for a 3T scanner. As a result, scan disruptive events may be reduced, and scanner use may become more efficient in departments with several scanners, which is very common.

In an example, the recommendation module 40 may be configured to recommend a relaxation technique, such as music, breathing, etc. A second evaluation may be done to see whether the recommended relaxation technique reduces the level of anxiety.

In an example, the recommendation module 40 may be configured to provide indication of the suitability of a patient to be assigned to a specific level of autonomy in a medical scanning procedure. In other words, the recommendation module 40 may determine whether the patient is a right choice for autonomous imaging or would require presence of support e.g. based on the ability of the patient to stay still during the simulated scan procedure. For example, if the patient has body movements up to a certain magnitude of displacement during the simulated scan procedure, which can be corrected by the MR itself using prospective motion compensation techniques. The patient is a right choice for autonomous imaging. Otherwise, staff support and/or support devices may be required at an interaction point in the scan where the patient is highly stressed.

In an example, the recommendation module 40 may be configured to determine whether a support device is required for an autonomous scan. For example, breath hold imaging in MRI is a technique with one or more stoppage of breathing during the sequence and require therefore a short scan time. Breath hold techniques are used with fast gradient echo sequences in thoracic or abdominal regions with much respiratory movement. Breath hold imaging requires the full cooperation of the patient, caused by usual MRI scan times from 15 to 20 sec. In the simulated scan procedure, if it is determined that the patient cannot hold the breath according to the requirement, an external breath hold assistance may be provided. For example, the administration of oxygen can help the patient to hold the breath during the scan. For example, during the simulated scan procedure, if the patient may have the urge to release stress or anxiety via body movements above a certain magnitude of displacement, which can be corrected by the MR itself using prospective motion compensation techniques, a patient positioning device may be required.

In an example, the recommendation module 40 may be configured to select a proper RF coil based on the patient profile for an autonomous setup. Dedicated coils may be recommended for imaging. Patient may be classified to need extra help for autonomous coil setting. Appropriate coils (size, weight, diameter, space, flexibility, cable or wireless) may be selected to fit to the profile (stress and/or anxiety level) of the patient.

In an example, the recommendation module 40 may be configured to select parameters, such as type of therapy mask, selection and adaptation of therapy settings, for a therapy system like MR-Linac/MRHifu based on the created patient profile.

In an example, the recommendation module 40 may provide an AR based patient guidance during the actual patient scan process based on a virtual environment scan process feedback.

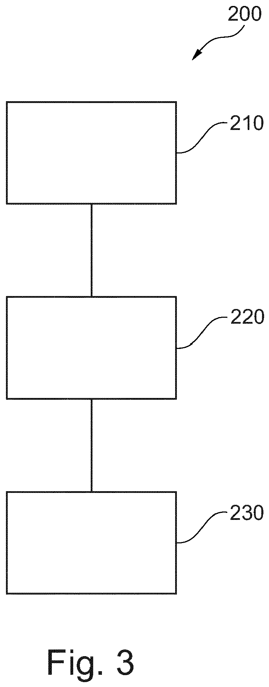

Fig. 3 illustrates a flowchart of a method 200 for patient profile creation.

In a first step 210, at least one sensory stimulus is applied over a patient to simulate a scan environment that may be experienced by a patient during a scan procedure.

The scan simulation module may provide a virtual environment of a scan and the feeling during the scan procedure to the patient in order to measure patient's reaction.

For example, a visual stimulation device, such as an HMD, may be used to provide the virtual environment of the scan. For example, a tactile stimulation device (e.g. a vibration device) and an auditory stimulation device (e.g. a binaural audio system) may be used to simulate further unpleasant aspects of the scans, such as scanner noise and vibration.

In a second step 220, data of the patient in the simulated scan environment is acquired.

In an example, a camera may be used to acquire image data of the patient. From the image information, several parameters of the imaged patient may be derived, such as heart rate, respiration rate, motion, facial expression, and pupil size (diameter).

In an example, a physiological sensor may be used to acquire physiological data of the patient. For example, the physiology sensor may be a temperature sensor to measure the patient's skin temperature, or a conductivity sensor to measure the electrical conductivity of the patient's skin.

In a third step 230, a state of anxiety of the patient is based on the acquired data and creating a patient profile comprising the determined state of anxiety of the patient in the simulated scan environment. This patent profile may be used to modify the scan stages or measure safety thresholds, for each of the patients, assess the level of autonomy the patient can be subjected to during the scan procedure and use the profile for making decisions during autonomous scanning.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one. "

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A system (100) for patient profile creation, comprising:
- a scan simulation module (10) comprising one or more sensory stimulation devices configured to apply at least one sensory stimulus over a patient to simulate a scan environment that may be experienced by a patient during a scan procedure;
- a patient monitoring module (20) comprising one or more sensors configured to acquire data of the patient in the simulated scan environment; and
- a patient profile generation module (30) configured to determine a state of anxiety of the patient based on the acquired data and to create a patient profile comprising the determined state of anxiety of the patient in the simulated scan environment.

2. System according to claim 1,
wherein the patient profile generation module is further configured to determine one or more of the following parameters based on the acquired data:
- a reason of anxiety; and
- a time instant at which the patient has a high level of anxiety during the scan procedure;
wherein the patient profile further comprises the one or more determined parameters.

3. System according to claim 1 or 2,
wherein the patient monitoring module comprises a sensor configured to monitor a behavior of the patient during the simulated scan procedure; and
wherein the patient profile generation module is configured to determine whether the patient acts in a compliant way with the simulated scan environment based on the monitored behaviour of the patient.

4. System according to any one of the preceding claims,
wherein the scan simulation module is configured to modify the at least one applied sensory stimulus to simulate a plurality of scan environments that may be experienced by the patient;
wherein the patient monitoring module is configured to acquire data of the patient in the plurality of simulated scan environments; and
wherein the created patient profile comprises the determined state of anxiety of the patient in the plurality of simulated scan environments.

5. System according to claim 4,
wherein the plurality of scan environments are different from each other in one or more of:
- a scanner type to be used;
- a level of autonomy of the scan procedure;
- a machine specification of a scanner; and
- a scan protocol.

6. System according to any one of the preceding claims,
wherein the scan simulation module comprises one or more of the following sensory stimulation devices:
- a visual stimulation device ;
- a tactile stimulation device; and
- an auditory stimulation device.

7. System according to any one of the preceding claims,
wherein the scan simulation module is configured to create an immersive environment.

8. System according to any one of the preceding claims,
wherein the patient monitoring module comprises one or more of the following sensors:
- a camera to acquire image data of the patient; and
- a physiology sensor configured to acquire physiological data of the patient.

9. System according to any one of the preceding claims, further comprising:
- a patient training module (50) configured to provide instructions to guide the patient through the scan procedure, and/or to educate the patient to identify a relevant set of feedback communication methods to provide the feedback during the scan procedure.

10. System according to any one of the preceding claims, further comprising:
- a recommendation module (40) configured to generate a personalized recommendation for an actual scan procedure for the patient based on the created patient profile.

11. System according to claim 10,
wherein the personalized recommendation comprises one or more of:
- selection of a scan protocol;
- selection of a scanner type;
- selection of a relaxation technique for the patient;
- indication of the suitability of a patient to be assigned to a specific level of autonomy in a medical scanning procedure;
- selection of a support device required for an autonomous scan;
- selection of a parameter for a therapy system; and
- provision of an augmented reality, AR, based patient guidance for an actual patient scan process.

12. System according to any one of the preceding claims,
wherein the system is a head-mounted system.

13. A method (200) for patient profile creation, comprising:
- applying (210) at least one sensory stimulus over a patient to simulate a scan environment that may be experienced by a patient during a scan procedure;
- acquiring (220) data of the patient in the simulated scan environment; and
- determining (230) a state of anxiety of the patient based on the acquired data and creating a patient profile comprising the determined state of anxiety of the patient in the simulated scan environment.

14. A computer program element for controlling a system according to any one of claims 1 to 12, which when being executed by a processor is configured to carry out the method according to claim 13.

15. A computer readable medium comprising the computer program element of claim 14.
